# EUROPEAN PATENT APPLICATION

(11) **EP 1 767 199 A2**
(43) Date of publication of application: **28.03.2007**
(21) Application number: 06126241.6
(22) Date of filing: 22.04.2003
(51) Int. Cl.: A61K 31/196, C07C 229/42

(54) **Methods and compositions for treatment of cancer pain**

(30) Priority: 23.04.2002 GB 0209257
(62) Divisional of application: 03725069.3
(71) Applicant: Novartis AG, 4056 Basel (CH); Novartis Pharma GmbH, 1230 Vienna (AT)
(72) Inventor: Fujimoto, Roger Aki, Winchester, 01890 (US); Urban, Laszlo, c/o Novartis Inst. for BioMed Rsrch, Cambridge, MA 02139 (US); Gonzalez, Isabel, Harlow, Essex CM19 5AW (GB)
(74) Representative: Leon, Susanna Iris

(57) **Abstract**

The invention provides a method of treating cancer pain, e.g. bone cancer pain, in a subject in need of such treatment which comprises administering to the subject an effective amount of a COX-2 inhibitor; advantageously a compound of formula I 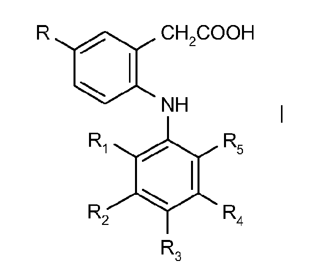 wherein R, R₁, R₂, R₃, R₄, and R₅ are as defined; a pharmaceutically acceptable salts thereof; or a pharmaceutically acceptable prodrug ester thereof.

## Description

This invention relates to selective cyclooxygenase-2 inhibitors (COX-2 Inhibitors), in particular to the use of COX-2 inhibitors in the treatment of cancer pain, especially bone cancer pain.

COX-2 inhibitors and their use as non-steroidal anti-inflammatory drugs (NSAIDs) for the treatment of inflammatory diseases and pain are well known in the art. Further it has been proposed (WO 99/11605) that COX-2 inhibitors may be useful for treatment of neoplasia particularly neoplasias that produce prostaglandins or express cyclooxygenase, including both benign and cancerous tumors, growths and polyps.

It has now been found that certain COX-2 inhibitors, in particular 5-alkyl substituted 2-arylaminophenylacetic acid derivative COX-2 inhibitors, have desirable properties for use in the treatment of Cancer pain, in particular bone cancer pain.

Accordingly the invention provides a method of treating cancer pain in a subject in need of such treatment, which comprises administering to the subject an effective amount of a COX-2 inhibitor.

Suitable COX-2 inhibitors for use in the invention may include the following compounds or derivatives thereof or a pharmaceutically acceptable salt thereof, or any hydrate thereof: rofecoxib, etoricoxib, celecoxib, valdecoxib, parecoxib, or a 5-alkyl-2-arylaminophenylacetic acid derivative COX-2 inhibitor.

In preferred embodiments the invention provides a method of treating cancer pain in a subject in need of such treatment which comprises administering to the subject an effective amount of a COX-2 inhibitor of formula I 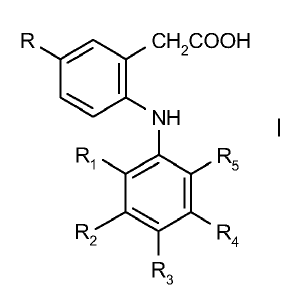 wherein R is methyl or ethyl;
R₁ is chloro or fluoro;
R₂ is hydrogen or fluoro;
R₃ is hydrogen, fluoro, chloro, methyl, ethyl, methoxy, ethoxy or hydroxy;
R₄ is hydrogen or fluoro; and
R₅ is chloro, fluoro, trifluoromethyl or methyl;
a pharmaceutically acceptable salt thereof; or
a pharmaceutically acceptable prodrug ester thereof.

Further the invention provides the use of a compound of formula I (or pharmaceutically acceptable salt or prodrug ester thereof) as defined above for the preparation of a medicament, for use in the treatment of cancer pain.

In a further aspect the invention provides use of a Cox-2 inhibitor, advantageously a compound of formula I (or pharmaceutically acceptable salt or prodrug ester thereof) as defined above for the treatment of cancer pain.

In a yet further aspect the invention provides cancer pain treatment agent comprising a Cox-2 inhibitor, advantageously a compound of formula I (or pharmaceutically acceptable salt or prodrug ester thereof) as defined above as active ingredient.

In a still yet further aspect the invention provides a package comprising a Cox-2 inhibitor, advantageously a compound of formula I (or pharmaceutically acceptable salt or prodrug ester thereof) as defined above together with instructions for use in the treatment of cancer pain.

The compounds of formula I may be used for treatment of cancer pain in general. In a particularly preferred embodiment the compounds of formula I are used for the treatment of bone cancer pain including pain associated with primary bone cancer such osteosarcoma, and also pain associated with bone metastases of primary cancers such as breast, colon, lung, prostate and other cancers, e.g. multiple myeloma.

In the present description the terms "treatment" or "treat" refer to both prophylactic or preventative treatment as well as curative or disease modifying treatment, including treatment of patients at risk of contracting the disease or suspected to have contracted the disease as well as patients who are ill or have been diagnosed as suffering from a disease or medical condition.

Particularly preferred compounds of formula I are those wherein R is methyl or ethyl; R₁ is chloro or fluoro; R₂ is hydrogen; R₃ is hydrogen, fluoro, chloro, methyl or hydroxy; R₄ is hydrogen; and R₅ is chloro, fluoro or methyl; pharmaceutically acceptable salts thereof; and pharmaceutically acceptable esters thereof.

A particularly preferred embodiment relates to the compounds of formula I wherein R is methyl or ethyl; R₁ is fluoro; R₂ is hydrogen; R₃ is hydrogen, fluoro or hydroxy; R₄ is hydrogen; and R₅ is chloro; pharmaceutically acceptable salts thereof; and pharmaceutically acceptable prodrug esters thereof.

Another particularly preferred embodiment of the invention relates to compounds of formula I wherein R is ethyl or methyl; R₁ is fluoro; R₂ is hydrogen or fluoro; R₃ is hydrogen, fluoro, ethoxy or hydroxy; R₄ is hydrogen or fluoro; and R₅ is chloro, fluoro or methyl; pharmaceutically acceptable salts thereof; and pharmaceutically acceptable prodrug esters thereof.

Further are said compounds wherein R is methyl or ethyl; R₁ is fluoro; R₂-R₄ are hydrogen or fluoro; and R₅ is chloro or fluoro; pharmaceutically acceptable salts thereof; and pharmaceutically acceptable prodrug esters thereof.

A further embodiment of the invention relates to the compounds of formula I wherein R is methyl or ethyl; R₁ is fluoro; R₂ is fluoro; R₃ is hydrogen, ethoxy or hydroxy; R₄ is fluoro; and R₅ is fluoro; pharmaceutically acceptable salts thereof; and pharmaceutically acceptable prodrug esters thereof.

Another embodiment of the invention relates to the compounds of formula I wherein R is methyl; R₁ is fluoro; R₂ is hydrogen; R₃ is hydrogen or fluoro; R₄ is hydrogen; and R₅ is chloro; pharmaceutically acceptable salts thereof; and pharmaceutically acceptable prodrug esters thereof. Particularly preferred embodiments of the invention relate to compounds of formula I
(a) wherein R is methyl; R₁ is fluoro; R₂ is hydrogen; R₃ is hydrogen; R₄ is hydrogen; and R₅ is chloro; pharmaceutically acceptable salts thereof; and pharmaceutically acceptable prodrug esters thereof;
(b) wherein R is methyl; R₁ is fluoro; R₂ is hydrogen; R₃ is fluoro; R₄ is hydrogen; and R₅ is chloro; pharmaceutically acceptable salts thereof; and pharmaceutically acceptable prodrug esters thereof;
(c) wherein R is ethyl; R₁ is fluoro; R₂ is fluoro; R₃ is hydrogen; R₄ is fluoro; and R₅ is fluoro; pharmaceutically acceptable salts thereof; and pharmaceutically acceptable prodrug esters thereof; and
(d) wherein R is ethyl; R₁ is chloro; R₂ is hydrogen; R₃ is chloro; R₄ is hydrogen; and R₅ is methyl; pharmaceutically acceptable salts thereof; and pharmaceutically acceptable prodrug esters thereof.

Most preferably 5-methyl-2-(2'-chloro-6'-fluoroanilino)-phenylacetic acid or a pharmaceutically acceptable salt thereof, or a pharmaceutically acceptable prodrug thereof is used as the COX-2 inhibitor of the invention.

Pharmacologically acceptable salts of the compounds of formula I are preferably salts with bases, conveniently metal salts derived from groups Ia, Ib, IIa and IIb of the Periodic Table of the Elements, including alkali metal salts, e.g. potassium and especially sodium salts, or alkaline earth metal salts, preferably calcium or magnesium salts, and also ammonium salts with ammonia or organic amines.

Pharmaceutically acceptable prodrug esters of the compounds of formula I are ester derivatives which are convertible by solvolysis or under physiological conditions to the free carboxylic acids of formula I. Such esters are e.g. lower alkyl esters (such as the methyl or ethyl ester), carboxy-lower alkyl esters such as the carboxymethyl ester, nitrooxy-lower alkyl esters (such as the 4-nitrooxybutyl ester), and the like. Preferred prodrugs are the compounds of formula Ia 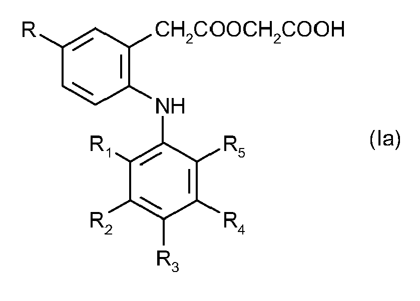 wherein R and R₁-R₅ have meaning as defined hereinabove for compounds of formula I; and pharmaceutically acceptable salts thereof.

Compounds of formula I and Ia and their synthesis are described in published international patent applications Nos. WO 99/11605 and WO 01/23346, the teachings of which are incorporated herein by reference.

It has been further discovered in accordance with the present invention that compounds of formual I (and esters and prodrugs thereof), in particular the compound 5-methyl-2-(2'-chloro-6'-fluoroanilino)-phenylacetic acid or a pharmaceutically acceptable salt thereof, or a pharmaceutically acceptable prodrug thereof, inhibit the radiologically observed structural changes in an animal model of bone cancer pain.

Thus in a yet further embodiment the invention includes a method for the inhibition of bone loss, advantageously in cancer, which comprises administering an effective amount of a COX-2 inhibitor of formula I (or an ester or prodrug thereof) as defined above to the subject in need of such treatment.

The Agents of the Invention, i.e. the COX-2 inhibitors of formula I and pharmaceutically acceptable salts and esters thereof , are preferably used in the form of pharmaceutical preparations that contain the relevant therapeutically effective amount of active ingredient optionally together with or in admixture with inorganic or organic, solid or liquid, pharmaceutically acceptable carriers which are suitable for administration.

The COX-2 pharmaceutical compositions may be, for example, compositions for enteral, such as oral, rectal, aerosol inhalation or nasal administration; compositions for parenteral, such as intravenous or subcutaneous administration; compositions for transdermal administration (e.g. passive or iontophoretic); or compositions for topical adminstration.

Preferably, the COX-2 pharmaceutical compositions are adapted to oral or parenteral (especially oral) administration. Intravenous and oral, first and foremost oral, adminstration is considered to be of particular importance. Preferably the COX-2 inhibitor active ingredient is in oral form.

The particular mode of administration and the dosage may be selected by the attending physician taking into account the particulars of the patient, especially age, weight, life style, activity level, etc.

The dosage of the Agents of the Invention may depend on various factors, such as effectiveness and duration of action of the active ingredient, mode of administration, warm-blooded species, and/or sex, age, weight and individual condition of the warm-blooded animal.

More particularly, the pharmaceutical compositions comprise an effective cyclooxygenase-2 inhibiting amount of COX-2 inhibitor or compound of formula I which is substantially free of cyclooxygenase-1 inhibiting activity and of side effects attributed thereto, when used therapeutically.

The compounds of formula I (and salts and esters thereof) are useful in the manufacture of pharmaceutical compositions comprising an effective amount thereof in conjunction or admixture with excipients or carriers suitable for either enteral or parenteral application. Preferred are tablets and gelatin capsules comprising the active ingredient together with a) diluents, e.g. lactose, dextrose, sucrose, mannitol, sorbitol, cellulose and/or glycine; b) lubricants, e.g. silica, talcum, stearic acid, its magnesium or calcium salt and/or polyethyleneglycol; for tablets also c) binders e.g. magnesium aluminum silicate, starch paste, gelatin, tragacanth, methylcellulose, sodium carboxymethylcellulose and or polyvinylpyrrolidone; if desired d) disintegrants, e.g. starches, agar, alginic acid or its sodium salt, or effervescent mixtures; and/or e) absorbents, colorants, flavors and sweeteners. Injectable compositions are preferably aqueous isotonic solutions or suspensions, and suppositories are advantageously prepared from fatty emulsions or suspensions. Said compositions may be sterilized and/or contain adjuvants, such as preserving, stabilizing, wetting or emulsifying agents, solution promoters, salts for regulating the osmotic pressure and/or buffers. In addition, they may also contain other therapeutically valuable substances. Said compositions are prepared according to conventional mixing, granulating or coating methods, respectively, and contain about 0.1 to 80%, preferably about 1 to 60% or more, of the active ingredient.

Tablets may be either film coated or enteric coated according to methods known in the art.

Suitable formulations for transdermal application include an effective amount of a compound of the invention with carrier. Advantageous carriers include absorbable pharmacologically acceptable solvents to assist passage through the skin of the host. For example, transdermal devices are in the form of a bandage comprising a backing member, a reservoir containing the compound optionally with carriers, optionally a rate controlling barrier to deliver the compound of the skin of the host at a controlled and predetermined rate over a prolonged period of time, and means to secure the device to the skin.

Suitable formulations for topical application, e.g. to the skin and eyes, include aqueous solutions, suspensions, ointments, creams, gels or sprayable formulations, for example, for delivery by aerosol or the like. Such topical delivery systems will in particular be appropriate for dermal application, e.g. for the treatment of skin cancer, for example, for prophylactic use in creams, lotions sprays and the like

The dosage of COX-2 inhibitor administered is dependent on the species of warm-blooded animal (mammal), the body weight, age and individual condition, and on the form of administration. A unit dosage for oral administration to a mammal of about 50 to 70 kg may contain between about 5 and 1500 mg, e.g. from 100-1000 mg, preferably 200-800 mg of the active ingredient.

COX-2 inhibitor formulations in single dose unit form contain preferably from about 1% to about 90%, and formulations not in single dose unit form contain preferably from about 0.1 % to about 20%, of the active ingredient. Single dose unit forms such as capsules, tablets or dragées contain e.g. from about 1mg to about 1000mg of the active ingredient.

COX-2 inhibitor pharmaceutical preparations for enteral and parenteral administration are, for example, those in dosage unit forms, such as dragées, tablets or capsules and also ampoules. They are prepared in a manner known *per se*, for example by means of conventional mixing, granulating, confectioning, dissolving or lyophilising processes. For example, pharmaceutical preparations for oral administration can be obtained by combining the active ingredient with solid carriers, where appropriate granulating a resulting mixture, and processing the mixture or granulate, if desired or necessary after the addition of suitable adjuncts, into tablets or dragée cores.

Parenteral formulations are especially injectable fluids that are effective in various manners, such as intravenously, intramuscularly, intraperitoneally, intranasally, intradermally or subcutaneously. Such fluids are preferably isotonic aqueous solutions or suspensions which can be prepared before use, for example from lyophilised preparations which contain the active ingredient alone or together with a pharmaceutically acceptable carrier. The pharmaceutical preparations may be sterilised and/or contain adjuncts, for example preservatives, stabilisers, wetting agents and/or emulsifiers, solubilisers, salts for regulating the osmotic pressure and/or buffers.

The following examples are intended to illustrate the invention and are not to be construed as being limitations thereon.

### EXAMPLES

### A. Formulation Examples

### Example 1

**Table 1**

| **Ingredient** | **Amount per 200 mg tablet batch (kg)** |
|---|---|
| **Core** | |
| **Granulation** | |
| 5-methyl-2-(2'-chloro-6'-fluoroanilino)phenylacetic acid drug substance | 50** |
| Microcrystalline cellulose, NF (PH 101) | 12.85 |
| Lactose monohydrate, NF | 11.65 |
| Croscarmellose sodium, NF | 1 |
| Povidone, USP | 4 |
| Titanium dioxide, USP | 2 |
| Water, purified ***, USP | 20.375 |

| **Extra-granular Phase** | |
|---|---|
| Microcrystalline cellulose, NF (PH 102) | 13 |
| Croscarmellose sodium, NF | 3 |
| Titanium dioxide, USP | 2 |
| Magnesium stearate, NF | 0.5 |

| **Coating** | |
|---|---|
| Opadry white | 2.801 **** |
| Opadry yellow | 2.0 **** |
| Opadry red | 0.4 **** |
| Opadry black | 0.0504 **** |
| Water, purified ***, USP | 29.758 **** |

| | |
|---|---|
| ** The weight of drug substance is taken with reference to the dried substance (100 per cent) on the basis of the assay value (factorization). The difference in weight is adjusted by the amount of microcrystalline cellulose used. *** Removed during processing. **** Includes a 50 % excess for loss during the coating process. | |

Table 1, above, sets out the formula for a batch of approximately 250,000 immediate release film-coated tablets of 5-methyl-2-(2'-chloro-6'-fluoroanilino)-phenylacetic acid. To make the tablets, titanium dioxide is dispersed in water, followed by the addition of povidone and mixing for 20 minutes to make a povidone/titanium dioxide suspension. The drug substance, lactose, microcrystalline cellulose, and croscarmellose are mixed in a high shear mixer (e.g., a Collette Gral) for 5 minutes to form a drug mixture. The drug mixture is granulated in the high shear mixer with the povidone/titanium dioxide suspension. The suspension is pumped at a rate of 3 kg/min into the drug mixture. The resulting mixture is mixed an additional 90 seconds after all the suspension is added. The wet granulation is dried in a fluid bed dryer, using an inlet air temperature of 50 °C. The residual water target is 3.5 % (with a permissible range of 2.5 - 4.5 %). The dried granulation is passed through a screen using a mill (oscillator) and a 30 mesh screen. The previous steps are repeated to make a second granulation.

The extra-granular phase titanium dioxide is passed through a 60 mesh hand screen. The dry granulations are mixed with the extra-granular phase microcrystalline cellulose, croscarmellose sodium and titanium dioxide in a twin shell mixer for 300 revolutions to form a penultimate mixture. Magnesium stearate is passed through a 60 mesh hand screen and is mixed with the penultimate mixture in a twin shell mixer for 50 revolutions to form a tableting mixture. The tableting mixture is pressed into tablets using a tablet press and oval punches.

The coating powders (Opadry) are mixed with purified water to make a 15 % w/w coating suspension. The tablets are film coated with the coating suspension in a coating pan using 60 °C to 75 °C inlet air temperature.

Table 2 sets out the contents of a 200 mg 5-methyl-2-(2'-chloro-6'-fluoroanilino)phenylacetic acid film-coated tablet.

**Table 2**

| **Ingredient** | **Theoretical amount [mg]** | **Function** |
|---|---|---|
| **Core** | | |
| 5-methyl-2-(2'-chloro-6'-fluoroanilino)phenylacetic acid drug substance | 200 | Active substance |
| Microcrystalline cellulose (PH 101) | 51.4 | Filler |
| Lactose | 46.6 | Filler |
| Povidone | 16 | Binder |
| Titanium dioxide | 8 | Color |
| Croscarmellose sodium | 4 | Disintegrant |
| Water, purified * | Q.S. | Granulating liquid |

| **Extragranular phase** | | |
|---|---|---|
| Microcrystalline cellulose (PH 102) | 52 | Filler |
| Croscarmellose sodium | 12 | Disintegrant |
| Titanium dioxide | 8 | Color |
| Magnesium stearate | 2 | Lubricant |
| Core weight | **400** | |

| **Coating** | | |
|---|---|---|
| Opadry white (00F18296) | 7.4676 | Color |
| Opadry yellow (00F12951) | 5.3312 | Color |
| Opadry red (00F15613) | 1.0668 | Color |
| Opadry black (00F17713) | 0.1344 | Color |
| Water, purified * | Q.S. | Coating solvent |
| **Total weight** | **414** | |

| | | |
|---|---|---|
| * removed during processing | | |

In addition, the tablet formulations may contain 5-methyl-2-(2'-chloro-6'-fluoroanilino)benzyl alcohol and/or 5-methyl-2-(2'-chloro-6'-fluoroanilino)benzoic acid in an amount between about 0.01 and 2% by weight, more specifically between about 0.1 and 1.

### Example 2

**Wet granulated tablet composition**

| Amount per tablet | Ingredient |
|---|---|
| 25 mg | COX-2 inhibitor |
| 79.7 mg | Microcrystalline cellulose |
| 79.7 mg | Lactose monohydrate |
| 6 mg | Hydroxypropyl cellulose |
| 8 mg | Croscarmellose sodium |
| 0.6 mg | Iron oxide |
| 1 mg | Magnesium stearate |

Tablet dose strengths of between 5 and 125 mg can be accomodated by varying total weight, and the ratio of the first three ingredients. Generally it is preferable to maintain a 1:1 ratio for microcrystalline cellulose: lactose monohydrate.

### Example 3

**Directly compressed tablet composition**

| Amount per tablet | Ingredient |
|---|---|
| 25 mg | COX-2 inhibitor |
| 106.9 mg | Microcrystalline cellulose |
| 106.9 mg | Lactose anhydrate |
| 7.5 mg | Croscarmellose sodium |
| 3.7 mg | Magnesium stearate |

Tablet dose strengths of between 5 and 125 mg can be accomodated by varying total tablet weight, and the ratio of the first three ingredients. Generally it is preferable to maintain a 1:1 ratio for microcrystalline cellulose:lactose monohydrate.

### Example 4

**Hard gelatine capsule composition**

| Amount per capsule | Ingredient |
|---|---|
| 25 mg | COX-2 inhibitor |
| 37 mg | Microcrystalline cellulose |
| 37 mg | Lactose anhydrate |
| 1 mg | Magnesium stearate |
| 1 capsule | Hard gelatin capsule |

Capsule dose strengths of between 1 and 50 mg can be accomodated by varying total fill weight, and the ratio of the first three ingredients. Generally it is preferable to maintain a 1:1 ratio for microcrystalline cellulose:lactose monohydrate.

### Example 5

**Oral solution**

| Amount per 5mL | Ingredient |
|---|---|
| 50 mg | COX-2 inhibitor |
| to 5 mL with Polyethylene oxide 400 | |

### Example 6

**Oral suspension**

| Amount per 5mL dose | Ingredient |
|---|---|
| 101 mg | COX-2 inhibitor |
| 150 mg | Polyvinylpyrrolidone |

**Oral suspension**

| Amount per 5mL dose | Ingredient |
|---|---|
| 2.5 mg | Poly oxyethylene sorbitan monolaurate |
| 10 mg | Benzoic acid |
| to 5 mL with sorbitol solution (70%) | |

Suspension dose strengths of between 1 and 50 mg/5 ml can be accomodated by varying the ratio of the first two ingredients.

### Example 7

**Intravenous infusion**

| Amount per 200 mL dose | Ingredient |
|---|---|
| 1 mg | COX-2 inhibitor |
| 0.2 mg | Polyethylene oxide 400 |
| 1.8 mg | Sodium chloride |
| to 200 mL | Purified water |

### Example 8 The Effect of COX-2 Inhibitors in a Rat Model of Bone Cancer pain

Adult female rats are given intra-tibial injections of MRMT-1 rat mammary gland carcinoma cells (3µl, 10⁷ cells/ml). These animals gradually develop mechanical hyperalgesia, mechanical allodynia (skin sensitivity to non-noxious stimuli) and hind limb sparing, beginning on day 12-14 following cell injection. COX-2 inhibitors, 5-methyl-2-(2'-chloro-6'-fluoroanilino)-phenylacetic acid (hereinafter referred to as COX189) (10 and 30 mg/kg p.o.) and valdecoxib (30 mg/kg p.o.) are administered as described below and the results obtained compared with vehcle treated controls.

Intra-tibial inoculation of MRMT-1 cells induces a time-dependent hypersensitivity in the ipsilateral limb, measured as a shift in body weight to the contralateral limb. The increase in weight-bearing difference between contralateral and ipsilateral paw (weight shift from the affected to the unaffected limb) is evident by day 14 post-inoculation and continues to increase until day 20.

The repeated administration of COX189 (10 and 30mg/kg, p.o.) from day 10 post-tumour cell inoculation significantly attenuates the weight-bearing difference on days 14, 17 and 20 measured 1 hour after drug administration. This effect is significant for COX189 on days 10, 14, 17 and 20 as well as for valdecoxib (30mg/kg, p.o.) on day 20. The doses chosen for COX189 and valdecoxib are comparable. Lower doses are not studied.

The effect is not dose-dependent, there is no difference between the effect of COX189 administered at doses of 10 and 30mg/kg. The treatments induces a progressive and long-lasting reduction in hypersensitivity, since the weight-bearing difference is also attenuated when measured 1h before the first daily treatment. This preventative effect reaches significant levels for all three treatments on days 14 and 17. There is no significant difference between the effects measured before and after COX189 administration on each day for any individual treatment.

There is pronounced mechanical allodynia in the ipsilateral paw (vehicle group) both to a punctuate stimulus (static allodynia) and to light stroking of the skin (dynamic allodynia) on day 20 post-inoculation. The repeated administration of COX189 (10 and 30mg/kg, p.o.) or valdecoxib (30mg/kg, p.o.) significantly reverses static allodynia on day 20, measured 90 minutes after the last administration. There is a tendency to reduce dynamic allodynia by all treatments, although this reduction only reaches statistical significance for COX189 at the highest dose (30mg/kg, p.o.).

Measurement of COX189 levels in serum of the same animals, taken 3 hours after administration on day 20, shows concentrations of mean±SEM:19.0±1.7µ.M and 59.9±3.9µM for COX189, 10 and 30mg/kg, respectively

The levels of thromboxane B₂ in the samples from COX189-treated animals are 87.4±17.7%, 64.5±8.0% and 77.4±27.8% of the levels in the vehicle-control animals, for COX189 (10mg/kg), COX189 (30mg/kg) and valdecoxib (30mg/kg), respectively. These data indicate that COX-1 is not inhibited by these doses, implying that the benefits seen in the model are COX-2 as opposed to COX-1 derived.

In addition it is found in this animal model experiment that repeated administration of COX189 at 10 and 30mg/kg significantly inhibits the radiologically observed structural changes on day 20. COX189 at 30mg/kg is able to significantly inhibit the loss of bone mineral density compared to vehicle treated animals. Valdecoxib at 30mg/kg has no significant effect on either parameter.

## Claims

1. A method of treating cancer pain in a subject in need of such treatment which comprises administering to the subject an effective amount of a COX-2 inhibitor;

2. A method according to claim 1 in which the COX-2 inhibitor is a compound of formula I 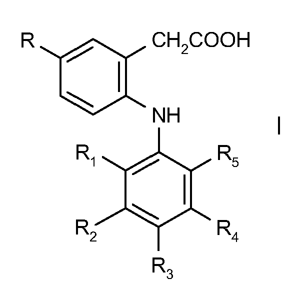 wherein R is methyl or ethyl;
R₁ is chloro or fluoro;
R₂ is hydrogen or fluoro;
R₃ is hydrogen, fluoro, chloro, methyl, ethyl, methoxy, ethoxy or hydroxy;
R₄ is hydrogen or fluoro; and
R₅ is chloro, fluoro, trifluoromethyl or methyl;
a pharmaceutically acceptable salts thereof; or
a pharmaceutically acceptable prodrug esters thereof..

3. Use of a compound of formula I as defined in claim 2 (or pharmaceutically acceptable salt or prodrug ester thereof) for the preparation of a medicament, for use in the treatment of cancer pain.

4. Use of a compound of formula I as defined in claim 2 (or pharmaceutically acceptable salt or prodrug ester thereof) for the treatment of cancer pain.

5. A package comprising a compound of formula I as defined in claim 2 (or pharmaceutically acceptable salt or prodrug ester thereof) together with instructions for use in the treatment of cancer pain.

6. A method according to claim 2, or use according to claim 3, in which the compound of formula I is 5-methyl-2-(2-chloro-6-fluoroanilino)-phenylacetic acid or a pharmaceutically acceptable salt thereof, or a pharmaceutically acceptable prodrug ester thereof.

7. A method according to claim 1 or use according to claim 3, for the treatment of bone cancer pain.

8. A method according to claim 1 or use according to claim 3, in which the compound of formula I is in the form of an oral composition or an injectable composition.

9. A method for the inhibition of bone loss, advantageously in cancer, which comprises administering an effective amount of a COX-2 inhibitor of formula I (or an ester or prodrug thereof) as defined in claim 2 to a subject in need of such treatment.

10. Use of a COX-2 inhibitor of formula I (or an ester or prodrug thereof) as defined in claim 2, for the preparation of a medicament for the inhibition of bone loss, in particular bone loss in cancer.
